# EUROPÄISCHE PATENTSCHRIFT

(11) **EP 2 251 636 B1**
(45) Veröffentlichungstag und Bekanntmachung des Hinweises auf die Patenterteilung: **05.06.2013**
(21) Anmeldenummer: 10004308.2
(22) Anmeldetag: 23.04.2010
(51) Int. Cl.: G01B 9/02, A61B 5/00, G01N 21/47

(54) **Vorwärtsscannendes OCT-Endoskop**
Forward-scanning OCT endoscope
Endoscope OCT à balayage en avant

(30) Priorität: 15.05.2009 DE 102009021580
(43) Veröffentlichungstag der Anmeldung: 17.11.2010
(73) Patentinhaber: Medizinisches Laserzentrum Lübeck GmbH, 23562 Lübeck (DE)
(72) Erfinder: Lankenau, Eva Dr., 23919 Rondeshagen (DE); Bonin, Tim, 22765 Hamburg (DE); Hüttmann, Gereon, 23564 Lübeck (DE)
(74) Vertreter: Appelt, Christian W.

(56) Entgegenhaltungen:
- WO-A1-2008/045851
- WO-A1-2008/137710
- DE-B3-102007 039 556
- US-A- 5 361 166
- US-A1- 2008 228 033
- U. SHARMA, N.M. FRIED, J.U. KANG: "All-Fiber Commo-Path Optical Coherence Tomography: Sensitivity Optimization and System Analysis" IEEE JOURNAL OF SELECTED TOPICS IN QUANTUM ELECTRONICS, Bd. 11, Nr. 4, 01545979, Juli 2005 (2005-07), Seiten 799-805, XP002592526

## Beschreibung

Die Erfindung betrifft eine Vorrichtung zur Optischen Kohärenztomographie (OCT) mit einem Applikator, der nach Art eines Endoskops zur Einführung in Hohlräume, insbesondere in Körperhohlräume eines lebenden Menschen oder Tieres, ausgebildet ist.

Das OCT-Verfahren ist heute Stand der Technik in der medizinischen Diagnostik. Dabei wird kurzkohärentes, breitbandiges Licht aus einer geeigneten Lichtquelle (z.B. aus einer Superlumineszendiode) zunächst in einen Proben- und einen Referenzlichtstrahl aufgeteilt, wonach der Probenstrahl auf das zu messende Objekt (z.B, lebendes Gewebe wie Epidermis, Retina) gerichtet und von diesem in verschieden Tiefen zurückgestreut wird. Das Referenzlicht wird hingegen grundsätzlich an einem Referenzspiegel reflektiert und derart geführt, dass es etwa dieselbe optische Weglänge wie das Probenlicht zurückgelegt hat, wenn es schließlich in einer Auswerteeinheit mit dem wiederkehrenden Probenlicht zusammengeführt und überlagert wird. Das interessierende Signal (die tiefenaufgelöste Streustärkenverteilung im Objekt) ergibt sich dann aus der Intensität des Interferenzlichts, das nur entsteht, wenn sich die Lichtwege von Proben- und Referenzstrahl um nicht mehr als die Kohärenzlänge des Lichts unterscheiden.

Bei der zeitabhängigen ("time domain", TD) OCT ist die Länge des Referenzarmes des Interferometers variabel ausgestaltet und wird gemeinhin mittels eines so genannten Phasenmodulators periodisch variiert. Zu definierten Zeitpunkten kann somit das Interferenzlicht nur von bestimmten Tiefen im Objekt herrühren. Die Realisierung und Kontrolle solcher Phasenmodulatoren ist technisch aufwendig und relativ teuer.

Vereinfachte OCT-Vorrichtungen, die mit einer festen Referenzarmlänge und somit ohne bewegte Teile auskommen, sind aus der WO 02/084263 A1 und aus der DE 43 09 056 A1 bekannt.

Das Verfahren der DE 43 09 056 A1 wird gemeinhin als "Spektralradar" oder auch als "Fourier domain" (FD-OCT) bezeichnet. Dabei wird Licht aus einer breitbandigen

Lichtquelle in der Probe in einer Ebene mit Abstand z zu einer Referenzebene (z=0) gestreut und mit zurück gestreutem Licht aus der Referenzebene überlagert. Es kommt so zu konstruktiver oder destruktiver Interferenz für einen beliebigen, festen Abstand z der Ebenen je nachdem, welche der eingestrahlten Wellenlängen λ man betrachtet. Das Interferenzlicht wird deshalb spektral zerlegt und üblicherweise auf eine Photodiodenzeile oder eine vergleichbare Vorrichtung abgebildet. Dies erlaubt das Messen der Verteilung I(k), k=2π/λ als räumliche Verteilung auf der Sensorzeile. Eine Fourier-Transformation dieser Verteilung führt auf das tiefenabhängige Streuvermögen S(z).

Bei der so genannten Linearen OCT (L-OCT) aus der WO 02/084263 A1 werden Proben- und Referenzlicht zunächst wie beim vorgenannten Spektralradar erzeugt und geführt und schließlich nach Art des bekannten Doppelspalt-Experiments räumlich überlagert. Das Interferenzlicht bildet ein Streifenmuster auf einem Zeilensensor (etwa ein CCD-Sensor), bei dem nun jedem Pixel des Sensors eine andere Laufzeit des Lichts - und damit eine andere Tiefe in der Probe - zugeordnet ist. Die Einhüllende der Intensitätsverteilung entlang der Sensorzeile enthält hier die Probeninformation.

Beide Verfahren zeichnen sich dadurch aus, dass der Referenzspiegel des Interferometers nahe an der Probe positioniert werden kann. Proben- und Referenzlicht können gleichzeitig über große Strecken in derselben Lichtleiterfaser geführt werden.

Dies ist von großem Vorteil, wenn das Probenlicht über einen beweglichen - eventuell in der Hand gehaltenen - Applikator auf das zu messende Objekt (meist ein Patient) eingestrahlt und von dort in eine stationäre Auswerteeinheit zurückgeführt werden soll. Jede Bewegung und/oder Verdrehung der Faser, aber auch Temperaturschwankungen oder mechanische Spannungen haben Einfluss auf die Laufzeit des Probenlichts und fuhren gegenüber einem Referenzarm, der diesen Faktoren nicht ausgesetzt ist, bei der Überlagerung von Proben- und Referenzlicht zu Störsignalen. Die gemeinsame Lichtführung in derselben Faser eliminiert dieses Problem.

Dies bedeutet aber, dass der Referenzspiegel ebenfalls im Applikator unterzubringen ist.

Gerade bei einem Applikator, der nach Art eines Endoskops verwendet werden soll, ist natürlich vorgegeben, dass der Applikator eine möglichst schmale, flexible, tubusartige Form aufweisen muss.

Ein Realisierungsvorschlag ist der WO 2009/140617 A2 oder auch der US 2008/228033 A1 zu entnehmen. In der zuletzt genannten Druckschrift ist vorgesehen, das distale Ende des Endoskops so auszugestalten, dass das aus der Lichtleiterfaser austretende Licht zunächst mittels einer Gradientenindex(GRIN)-Linse in das seitlich neben dem Endoskopende befindliche Objektmaterial (mithin organisches Gewebe) fokussiert wird. Dabei wird hinter der GRIN-Linse ein Strahlteiler angeordnet, der einen Strahlanteil durch ein an der Seite des Endoskopendes vorgesehenes Austrittsfenster in das Gewebe lenkt und das von dort wiederkehrende Licht durch die GRIN-Linse zurück in die Faser führt. Der zweite Strahlanteil wird beispielsweise in Vorwärtsrichtung des Endoskops zu einer verspiegelten Fläche durchgelassen, die im Sinne der OCT als Referenzspiegel dient. Alternativ wird auch vorgeschlagen, das seitliche Austrittsfenster selbst sowohl als Strahlteiler als auch als Referenzspiegel zu verwenden, etwa durch Vorsehen einer teilreflektierenden Beschichtung auf dem Austrittsfenster.

Der Vorschlag der US 2008/228033 A1 lässt sich gut in kompakter und vor allem schlanker Bauweise realisieren. Er hat allerdings den Nachteil, dass die OCT-Messung nur seitlich im unmittelbar dem Endoskop benachbarten Gewebe gelingen kann.

Wünschenswert wäre aber die Möglichkeit der OCT-Messung in Vorwärtsrichtung des Endoskops, was zugleich bedeuten kann, dass das zu untersuchende Objekt etliche Millimeter Abstand zum Austrittsfenster des distalen Endoskopendes aufweist. Zwar ist es unproblematisch, eine ausreichend große Brennweite der fokussierenden Optik einzurichten, aber das Vorsehen von Strahlteiler und Referenzspiegel im oder am nach vom gerichteten Austrittsfenster ist keine günstige Maßnahme, da sich Proben- und Referenzarmlänge des Interferometers dann zu sehr unterscheiden. Vielmehr muss ein angemessen langer Lichtlaufweg für das Referenzlicht vorgesehen werden, wobei der dann eigens erforderliche Referenzspiegel nicht in Vorwärtsrichtung des Endoskops angeordnet werden kann,

Nahe liegend und auch dem Stand der Technik zu entnehmen ist der Vorschlag, die Lichtführung der US 2008/228033 A1 hinter der fokussierenden Optik schlicht um 90° zu drehen, d.h. das Referenzlicht senkrecht zur Längsachse des Endoskops abzulenken und auf einen seitlich angeordneten Referenzspiegel zu führen. Wenn dieser seitlich gerichtete Referenzarm jedoch etliche Millimeter Länge benötigt, wird er entweder apparativ sehr kompliziert oder das distale Ende des Endoskops wird nicht sehr schmal.

Es ist gängig, in Endoskopen GRIN-Linsen zur Fokussierung zu verwenden. Diese weisen typisch ein radial verlaufendes, parabolisches Profil des Brechungsindex auf, was zur Folge hat, das eingestrahlte Lichtstrahlen innerhalb einer GRIN-Optik einen sinusförmigen Verlauf nehmen. Eine GRIN-Optik besitzt insofern eine intrinsische Periodenlänge, auch Pitch genannt Eine Linse der Länge 1 Pitch besitzt die Eigenschaft, dass ein auf der einen Seite mit beliebiger Richtung eingestrahlte Lichtstrahl die Optik auf der anderen Seite in exakt dieselbe Richtung verlässt. Das Licht einer Punktquelle auf der Einstrahloberfläche wird in einen Punkt der Austrahloberfläche fokussiert, der der Punktquelle genau gegenüber liegt.

Eine GRIN-Optik der Länge¼ Pitch hingegen kollimiert das Licht dieser Punktquelle.

Aus diesem Sachverhalt ergibt sich die Anwendbarkeit von GRIN-Optiken mit einer Länge von n/4 (n - ungerade ganze Zahl) Pitch als Retroreflektoren, wie z.B. in der US 4 789 219 nachzulesen ist.

Auch für interferometrische Messungen ist die Nutzung von GRIN-Optiken als Retroreflektoren bereits in der EP 1 647 798 A1 vorgeschlagen worden. In dieser Druckschrift geht es um ein interferometrisches Verfahren zur Positionsbestimmung von Oberflächen, deren Ausrichtung nicht vorab bekannt oder aber variabel ist. Das Probenlicht wird deshalb nicht senkrecht, sondern unter einem Verkippungswinkel, auf die Oberfläche des zu messenden Objektes eingestrahlt. Folglich kehrt das reflektierte Probenlicht nicht sofort in die Einstrahlrichtung zurück, sondern es bedarf eines nachgeordneten Retroreflektors, damit dies erreicht wird. Der Gedanke der EP 1 647 798 A1 liegt nun u. a darin, eine ¼ Pitch GRIN-Optik sowohl zur Kollimierung des aus einer Punktquelle (z.B. Monomodeglasfaser) eingestrahlten Lichts als auch zugleich als Retroreflektor im Probenarm zu verwenden. Der Referenzarm des Interferometers ist in allen Ausgestaltungen der Erfindung senkrecht zur Einstrahlrichtung orientiert (man beachte dort insbesondere die Fig. 4 A und B, die verschiedene Seitenansichten derselben Vorrichtung darstellen).

Es ist die Aufgabe der Erfindung, einen Applikator für ein OCT-System vorzuschlagen, der eine besonders kompakte und schmale Bauform aufweist und sich dadurch für die Verwendung nach Art eines Endoskops eignet, wobei er die OCT-Messung in Vorschubrichtung (i. F. auch vorwärts gerichtet) erlaubt.

Die Aufgabe wird gelöst durch eine Vorrichtung mit den Merkmalen des Hauptanspruchs. Die Unteransprüche geben vorteilhafte Ausgestaltungen an.

Der erfindungsgemäße Applikator umfasst eine Lichtleiterfaser, die nach dem Stand der Technik zur Führung des OCT-Lichts geeignet ist, in einem ebenfalls an sich bekannten flexiblen Tubus. Das distale Ende des Tubus wird erfindungsgemäß mit einer Anordnung in folgender Reihenfolge versehen: hinter dem Austrittsende der Lichtleiterfaser wird ein GRIN-Device angeordnet, hiernach eine fokussierende Optik und gegebenenfalls ein Austrittsfenster in Vorschubrichtung, wobei die fokussierende Optik zur Fokussierung des Lichts in Vorschubrichtung des Applikators eingerichtet ist.

Das GRIN-Device umfasst erfindungsgemäß eine an sich bekannte GRIN-Optik mit einer proximalen (faserseitigen) und einer distalen (der Faser gegenüberliegenden) Flachseite. Ein Spiegel wird auf oder unmittelbar vor der proximalen Flachseite in Nachbarschaft des Faseraustritts angeordnet. Ein Strahlteiler wird auf oder unmittelbar hinter der distalen Flachseite angeordnet. Besonders bevorzugt sind die Flachseiten der GRIN-Optik zu diesem Zweck mit Funktionsschichten versehen, insbesondere einer hochreflektierenden Schicht auf der proximalen Flachseite und einer teilreflektierenden Schicht auf der distalen Flachseite. Auf die genaue Ausgestaltung von Spiegel und Strahlteiler kommt es nicht an. Wesentlich ist allerdings dass die Spiegelebenen der Lichtreflexion durch Spiegel und Strahlteiler mit den Flachseiten der GRIN-Optik zusammenfallen.

Die Länge der GRIN-Optik stellt erfindungsgemäß im Wesentlichen die Länge des Referenzarmes des OCT-Systems dar. Da sie nun in Richtung der Längsachse des Tubus, d.h. in Vorschubrichtung, orientiert ist, sind relativ große Längen bei zugleich schmaler Bauform des Applikators möglich.

Die genaue Länge der GRIN-Optik ist der Wahl des Herstellers überlassen, wobei stets zu beachten ist, dass die Laufzeiten des Lichts in Proben- und Referenzarm des Interferometers im Wesentlichen gleich sein müssen, um eine OCT-Messung durchzuführen. Für die beiden Lichtwege vom Strahlteiler zum Referenzspiegel bzw. zur Probe muss also gelten, dass die Verhältnisse der Weglängen zu den jeweiligen Gruppengeschwindigkeiten des Lichts (ggf. summiert über Teilweglängen mit unterschiedlichen Grugpengeschwindigkeiten) im Wesentlichen übereinstimmen.

Das Indexprofil der GRIN-Optik ist nun erfindungsgemäß so einzurichten, dass die gewählte Länge der GRIN-Optik eine Pitchlänge von N/8 bildet, wobei N aus der Menge der natürlichen, nicht durch 4 teilbaren Zahlen zu wählen ist, Zur Verdeutlichung: N = 1, 2, 3, 5, 6, 7, 9, ... sind möglich.

Das GRIN-Device dieser Erfindung umfasst damit - vorzugsweise in baulicher Einheit - den Referenzspiegel des Interferometers, eine GRIN-Optik mit N/8-Pitch (N s. o.) bei beliebig gewählter Länge und den Strahlteiler des Interferometers. Nur das Licht, welches den Strahlteiler passiert, wird durch die fokussierende Optik in das Gewebe fokussiert und verlässt den Applikator durch das Austrittsende in Vorwärtsrichtung. Die fokussierende Optik befindet sich somit allein im Probenzweig des Interferometers. Insbesondere ist die GRIN-Optik des erfindungsgemäßen GRIN-Devices nicht Bestandteil der fokussierenden Optik. Gleichwohl kann die fokussierende Optik natürlich auch als GRIN-Linse ausgeführt sein, doch dies ist für die Erfindung unwichtig. Ebenso eignet sich jede andere Ausführung der Fokussierung, etwa eine konvex-plane Linse.

Die Erfindung wird nachfolgend anhand zweier Figuren näher erläutert. Dabei zeigt:
- Fig. 1: Den Strahlverlauf in einem erfindungsgemäßen Applikator, bei dem die Lichtleiterfaser zentriert in das GRIN-Device einstrahlt und N = 5 gesetzt ist;
- Fig. 2: Den Strahlverlauf in einem erfindungsgemäßen Applikator, bei dem die Lichtleiterfaser nicht zentriert in das GRIN-Device einstrahlt und N = 2 gesetzt ist;

Grundsätzlich macht sich die Erfindung die bildleitenden Eigenschaften von GRIN-Optiken zunutze, um den Referenzarm mit einfachen Mitteln zu realisieren. Dabei sind prinzipiell zwei Varianten möglich, je nachdem, ob eine achsensymmetrische Lichtführung realisiert werden soll oder nicht.

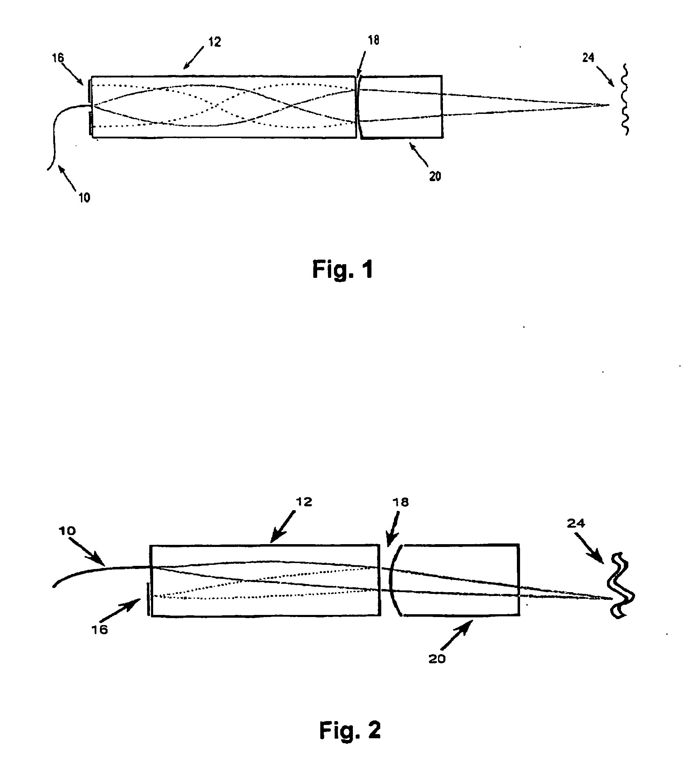

Fig. 1 stellt den symmetrischen Fall dar. Hierbei muss N eine ungerade Zahl sein, beispielhaft wird N = 5 gesetzt. Die Lichtleiterfaser 10 wird mit dem erfindungsgemäßen GRIN-Device 12 derart verbunden (i. a. verklebt), dass der Lichtaustritt aus der Faser genau im Zentrum der proximalen Flachseite entlang der Symmetrieachse des GRIN-Device erfolgt. Die proximale Flachseite ist vollständig verspiegelt mit Ausnahme der Umgebung des Faserendes, d.h. es ist eine im Zentrum unterbrochene, hochreflektierende Beschichtung 16 auf der proximalen Flachseite angeordnet. Die durchgezogenen Linien deuten den Strahlverlauf des vom Faseraustrittsende ausgehenden Lichtkegels an, der zunächst zur distalen Flachseite des GRIN-Devices führt, wo das Licht durch eine teilreflektierende Beschichtung 18 aufgeteilt wird. Der transmittierte Lichtanteil wird durch eine fokussierende Optik 20 (hier exemplarisch eine konvex-planare Linse) in Vorwärtsrichtung in das zu messende Objekt 24 fokussiert und von dort zurückgestreut. Das Probenlicht bzw. Streulicht nimmt hiernach denselben Weg zur Lichtleiterfaser 10 zurück. Der am Strahlteiler reflektierte Lichtanteil - das Referenzlicht - durchläuft erneut das GRIN-Device 12 (gepunktete Linie). Es hat also bei Erreichen der Spiegelfläche 16 konstruktionsbedingt eine zusätzliche, mit der Brennweite der fokussierenden Optik 20 korrespondierende Strecke zurückgelegt und dabei zugleich insgesamt eine 2*N/8 = N/4 Pitch GRIN-Optik mit N ungerade durchquert. Folglich wird es exakt in sich zurückgespiegelt, gelangt dabei abermals zur teilreflektierenden Schicht 18 und wird von dort aus anteilig wieder auf das Faseraustrittsende im Zentrum der proximalen Flachseite des GRIN-Device 12 fokussiert, zusammen mit dem vom Messobjekt 24 wiederkehrenden Probenlicht. Ein kleiner Anteil des Referenzlichts geht bei der Spiegelung an der unterbrochenen Schicht 16 verloren, da er in die Faser 10 gelangt, was unschädlich für die Auswertung ist. Ein größerer Anteil des Referenzlichts geht bei der zweiten Reflexion an Schicht 18 verloren, da er den Applikator nach vorne verlässt.

Fig. 2 zeigt den Fall asymmetrischer Strahlführung, bei dem N gerade, aber kein Vielfaches von 4, sein soll. Exemplarisch ist N = 2 gesetzt. Die Lichtleiterfaser 10 wird hierbei außerhalb der Symmetrieachse der GRIN-Optik angeordnet. Die erfindungsgemäße GRIN-Optik kollimiert das Licht aus der Faser 10. Der Austritt des kollimierten Lichts aus der distalen Flachseite des GRIN-Device 12 erfolgt immer unter einem Winkel ungleich Null gegen die Symmetrieachse der GRIN-Optik. Auch hier ist eine teilreflektierende Schicht 18 an der distalen Flachseite angeordnet. Der Lichtanteil, der vom Strahlteiler 18 aus in die GRIN-Optik zurückgeführt wird (auch hier das Referenzlicht), wird nun in einen Punkt auf der proximalen Seite der GRIN-Optik fokussiert. Dieser Fokuspunkt liegt in der proximalen Flachseite, benachbart zum Faseraustritt, aber diesem punktsymmetrisch zur Flachseitenmitte gegenüber. Es reicht daher aus, nur diese relativ kleine Fläche in der Nachbarschaft des Faseraustritts mit einer hochreflektierenden Schicht 16 zu belegen. Abermals fungiert die GRIN-Optik als Retroreflektor, und das Referenzlicht gelangt nach erneuter Reflektion am Strahlteiler 18 wieder zurück in die Faser 10. Für das Probenlicht gilt das zu Fig. 1 Gesagte.

An dieser Stelle lässt sich leicht erklären, warum N = 4, 8, 12, ... ungeeignet sind, denn dann würde der Referenzlichtstrahl bei zweimaligem Durchlaufen der GRIN-Optik direkt zurück in das Faserende fokussiert und wäre für die Auswertung im Interferometer verloren.

In den beiden zuvor beschriebenen Figuren ist kein Austrittsfenster dargestellt. Dem Fachmann ist klar, dass dieses z.B. mit der Flachseite der konvex-planaren Linse zusammenfallen oder aber auch ein zusätzliches Bauteil sein kann. Für die Erfindung spielt die Ausgestaltung des Austrittsfensters keine Rolle.

Eine vorteilhafte Ausgestaltung der teilreflektierenden Schicht (Strahlteiler18) liegt in der Beschichtung des GRIN-Device mit einem dielektrischen Material oder mit einem dünnen Metallfilm. Beispielsweise eignet sich ein dünner Goldfilm von wenigen Nanometern Dicke, vorzugsweise weniger als 20 nm.

Ebenfalls vorteilhaft ist auch eine geometrische Strahlteilung. Dazu wird die distale Flachseite des GRIN-Device komplett verspiegelt (etwa metallisiert, z.B. mit Gold) bis auf eine zentrale kreisförmige Apertur, die kleiner als der Strahldurchmesser ist. Somit wird der äußere Bereich des Modenfeldes reflektiert, wodurch eine geometrische Strahlteilung erfolgt.

## Patentansprüche

1. Vorrichtung für die optische Kohärenztomographie, mit einer kurzkohärenten, breitbandigen Lichtquelle und einer das Licht von der Lichtquelle zu einer fokussierenden Optik (20) leitenden Lichtleiterfaser (10),
**gekennzeichnet durch**
- eine zwischen Lichtleiterfaser (10) und fokussierender Optik (20) angeordnete Gradientenindex-Optik (12) mit zwei sich gegenüberliegenden parallelen Flachseiten, die an ihrer ersten Flachseite mit der Lichtleiterfaser (10) einen der Gradientenindex-Optik (12) Licht zuführenden Einstrahlpunkt bildend kontaktiert ist und einen Pitch von N/8 aufweist, wobei N eine natürliche, nicht durch 4 teilbare Zahl ist;
- ein auf der ersten Flachseite der Gradientenindex-Optik (12), dem Einstrahlpunkt benachbart angeordnetes als Referenzspiegel dienendes erstes Mittel (16) zur Lichtreflexion des Lichts; und
- ein auf der zweiten Flachseite der Gradientenindex-Optik (12) angeordnetes zweites Mittel (18) zur Strahlteilung des Lichts zur Erzeugung eines auf das erste Mittel reflektierten Referenzlichts und eines transmittierten Lichts,
wobei die fokussierende Optik (20) zur Fokussierung des **durch** das zweite Mittel (18) transmittierten Lichts senkrecht zu den Flachseiten der Gradientenindex-Optik (12) eingerichtet ist.

2. Vorrichtung nach Anspruch 1, **dadurch gekennzeichnet, dass** der Einstrahlpunkt im Zentrum der ersten Flachseite der Gradientenindex-Optik (12) liegt, das erste Mittel (16) die erste Flachseite mit Ausnahme des Einstrahlpunktes vollflächig abdeckt und N eine ungerade natürliche Zahl ist.

3. Vorrichtung nach Anspruch 1, **dadurch gekennzeichnet, dass** der Einstrahlpunkt außerhalb des Zentrums der ersten Flachseite der Gradientenindex-Optik (12) liegt, das erste Mittel (16) jenen Punkt der ersten Flachseite abdeckt, der sich durch Punktspiegelung des Einstrahlpunktes am Zentrum der ersten Flachseite ergibt und N eine gerade, nicht durch 4 teilbare natürliche Zahl ist.

4. Vorrichtung nach einem der vorangehenden Ansprüche, **dadurch gekennzeichnet, dass** das erste Mittel (16) auf der ersten Flachseite der Gradientenindex-Optik (12) als hochreflektierende erste Beschichtung ausgebildet ist.

5. Vorrichtung nach Anspruch 4, **dadurch gekennzeichnet, dass** die erste Beschichtung ein lichtundurchlässiger Metallfilm ist.

6. Vorrichtung nach einem der vorangehenden Ansprüche, **dadurch gekennzeichnet, dass** das zweite Mittel (18) auf der zweiten Flachseite der Gradientenindex-Optik (12) als teilreflektierende zweite Beschichtung ausgebildet ist.

7. Vorrichtung nach Anspruch 6, **dadurch gekennzeichnet, dass** die zweite Beschichtung ein Goldfilm mit einer Dicke kleiner als 20 nm ist.

8. Vorrichtung nach einem der Ansprüche 1 bis 5, **dadurch gekennzeichnet, dass** die zweite Flachseite mit Ausnahme eines frei bleibenden zentralen Bereichs, der kleiner als der Strahldurchmesser ist, mit einer hochreflektierenden Beschichtung verspiegelt ist.

## Claims

1. A device for optical coherence tomography, including a short-coherent, wide-band light source, and an optical fiber (10) guiding the light from the light source to a focusing optics (20), **characterized by**:
- a gradient-index optics (12) arranged between said optical fiber (10) and said focusing optics (20), said gradient-index optics (12) having two parallel flat faces opposite each other and contacting, on its first flat face, said optical fiber (10) to form an irradiation point feeding light to said gradient-index optics (12), and having a pitch of N/8, N being a natural number which is not divisible by four;
- first means (16) for reflecting the light, the first means (16) being arranged adjacent to the irradiation point on the first flat face of said gradient-index optics (12) to serve as a reference mirror; and
- second means (18) for dividing the light, the second means (18) being arranged on the second flat face of said gradient-index optics (12) to generate a reference light reflected onto said first means, and a transmitted light,
wherein said focusing optics (20) is adapted to focus the light transmitted through said second means (18) perpendicularly to the flat faces of said gradient-index optics (12).

2. The device according to claim 1, **characterized in that** the irradiation point is located in the center of the first flat face of said gradient-index optics (12), said first means (16) covers the whole area of the first flat face except for the irradiation point, and N is an odd natural number.

3. The device according to claim 1, **characterized in that** the irradiation point is located outside the center of the first flat face of said gradient-index optics (12), said first means (16) covers a point of the first flat face, which results from a point reflection of the irradiation point in the center of the first flat face, and N is an even natural number which is not divisible by four.

4. The device according to any one of the preceding claims, **characterized in that** said first means (16) are formed as a highly reflecting first coating on the first flat face of said gradient-index optics (12).

5. The device according to claim 4, **characterized in that** the first coating is an opaque metal film.

6. The device according to any one of the preceding claims, **characterized in that** said second means (18) are formed as a partially reflecting second coating on the second flat face of said gradient-index optics (12).

7. The device according to claim 6, **characterized in that** the second coating is a gold film having a thickness of less than 20 nm.

8. The device according to any one of claims 1 to 5, **characterized in that** said second flat face has a highly reflecting coating, except for an uncoated central portion which is smaller than the diameter of the beam.

## Revendications

1. Dispositif de tomographie optique cohérente comprenant une source lumineuse à large bande à cohérence courte et un guide de lumière par fibres optiques (10) dirigeant la lumière de la source lumineuse vers un objectif de focalisation (20), **caractérisé par** :
- un objectif à indice de gradient (12) agencé entre le guide de lumière par fibres optiques (10) et l'objectif de focalisation (20) comprenant deux côtés plats parallèles situés l'un en face de l'autre, qui au niveau de son premier côté plat, est en contact avec le guide de lumière par fibres optiques (10) en formant un point de radiation incidente amenant la lumière à l'objectif à indice de gradient (12) et qui présente un pas de N/8, étant entendu que N est un nombre naturel non divisible par 4 ;
- un premier moyen (16) de réflexion lumineuse de la lumière agencé sur le premier côté plat de l'objectif à indice de gradient (12), en position adjacente au point de radiation incidente, faisant fonction de miroir de référence ; et
- un deuxième moyen (18) agencé sur le deuxième côté plat de l'objectif à indice de gradient (12) destiné à séparer le faisceau de lumière afin de produire une lumière de référence se réfléchissant sur le premier moyen et une lumière de transmission,
étant entendu que l'objectif de focalisation (20) est installé perpendiculairement aux côtés plats de l'objectif à indice de gradient (12) afin de focaliser la lumière transmisse par le biais du deuxième moyen (18).

2. Dispositif selon la revendication 1, **caractérisé en ce que** le point de radiation incidente se situe au centre du premier côté plat de l'objectif à indice de gradient (12), le premier moyen (16) recouvre le premier côté plat sur toute sa surface à l'exception du point de radiation incidente et N est un nombre naturel impair.

3. Dispositif selon revendication 1, **caractérisé en ce que** le point de radiation incidente se situe à l'extérieur du centre du premier côté plat de l'objectif à indice de gradient (12), le premier moyen (16) recouvre le point du premier côté plat qui résulte de la réflexion du point de radiation incidente au centre du premier côté plat et N est un nombre naturel pair non divisible par 4.

4. Dispositif selon l'une des revendications précédentes, **caractérisé en ce que** le premier moyen (16) est réalisé en tant que premier revêtement hautement réfléchissant sur le premier côté plat de l'objectif à indice de gradient (12).

5. Dispositif selon la revendication 4, **caractérisé en ce que** le premier revêtement est un film métallique opaque.

6. Dispositif selon l'une des revendications précédentes, **caractérisé en ce que** le deuxième moyen (18) est réalisé en tant que deuxième revêtement partiellement réfléchissant sur le deuxième côté plat de l'objectif à indice de gradient (12).

7. Dispositif selon la revendication 6, **caractérisé en ce que** le deuxième revêtement est un film d'or d'une épaisseur inférieure à 20 nm.

8. Dispositif selon l'une des revendications 1 à 5, **caractérisé en ce que** le deuxième côté plat est métallisé par un revêtement hautement réfléchissant à l'exception d'une zone centrale restant dégagée, qui est plus petite que le diamètre du faisceau.
